# EUROPEAN PATENT APPLICATION

(11) **EP 1 550 458 A1**
(43) Date of publication of application: **06.07.2005**
(21) Application number: 03029801.2
(22) Date of filing: 23.12.2003
(51) Int. Cl.: A61K 39/39, A61K 9/127

(54) **Synergistic liposomal adjuvants**

(71) Applicant: Vectron Therapeutics AG, 35037 Marburg (DE)
(72) Inventor: Konur, Abdo, Dr., 35037 Marburg (DE); Graser, Andreas, 35041 Marburg (DE)
(74) Representative: Zwicker, Jörk

(57) **Abstract**

The present invention relates to liposome, mixtures or liposomes and liposomal compositions comprising at least two different adjuvants and a therapeutic agent, their production and use for the prevention and therapy of proliferative diseases, infectious diseases, vascular diseases, rheumatoid diseases, inflammatory diseases, immune diseases, in particular autoimmune diseases and allergies.

## Description

### Background of the Invention

### 1. Field of the Invention

The present invention relates to liposomes, mixtures or liposomes and liposomal compositions comprising at least two different adjuvants and a therapeutic agent, their production and use for the prevention and therapy of proliferative diseases, infectious diseases, vascular diseases, rheumatoid diseases, inflammatory diseases, immune diseases, in particular autoimmune diseases and allergies.

### 2. Description of Related Art

Vaccination strategies have been used for decades primarily to foster a protective immunity to protect patients from developing a disease after contact with an infectious agent. To this end live attenuated, dead or disrupted pathogens, pathogen preparations, or purified or recombinant components of the pathogens have been administered to patients to elicit a specific immune response to antigenic components of the respective pathogen. The components, which stimulate such an immune response can be, for example, pathogen specific proteins, polysaccharides or lipids. The specific immune response against antigens comprised within pathogens can be further stimulated by the coadministration of adjuvants. This was discovered by Ramon in the mid-1920s, when he observed that horses, which developed abscesses at the site of an injection of diphtheria toxin, produced higher antitoxin titers than animals without abscesses. Further evidence for the adjuvant effect of certain compounds was given by Glenny et al. (1926) J. Path. Bact. 29: 38-45, who showed that the immune response against diphtheria toxin could be enhanced by the coadministration of aluminum compounds. In the mid-1930 Freund developed a powerful immunologic adjuvant composed of a water-in-mineral oil emulsion, which comprised killed disrupted mycobacteria.

Thus, adjuvants are known in the art to accelerate, prolong, or enhance the quality of the specific immune response to the antigen or antigens and are currently employed in all licensed US-vaccines. The proposed advantages of adjuvants include their ability to: 1) direct and optimize immune responses that are appropriate for the vaccine; 2) enable mucosal delivery of vaccines; 3) promote cell-mediated immune response; 4) enhance the immunogenicity of weaker immunogens such as highly purified or recombinant antigens; 5) reduce the amount of antigen or the frequency of immunization required to provide protective immunity; 6) improve efficacy of vaccines in individuals with reduced or weakened immune responses such as newborns, the aged, and immunocompromized patients.

Adjuvants have diverse mechanisms of action. The first mechanism of adjuvant action identified was the so-called depot effect, in which gel-type adjuvants such as aluminum hydroxide (alum) or emulsion based adjuvants such as incomplete Freund's adjuvants (IFA) associate with antigens and facilitate transport of the antigen to the draining lymph nodes, where immune responses are generated. Immunologic adjuvants act directly or indirectly on antigen presenting cells (APCs) such as macrophages, Langerhans cells and dendritic cells (Wu J.Y. et al. (1994) Cell Immunol. 154: 393; Kovacsovics-Bankowski M. et al. (1994) 24: 2421). Some adjuvants have been shown to improve the induction of MHC class I restricted CD8+ cytotoxic T cell (CTL) response, if compared to standard alum adjuvants (Takhashi H. et al. (1990) Nature 344: 873; Newmann M.J. et al. (1992) J. Immunol. 148: 2357-2362; Shahum E. et al. (1995) Int. J. Immunopharmacol. 17: 9) possibly because these adjuvants might facilitate direct delivery of the antigen to the cytosol for presentation with MHC class I molecules. Antigen presented to the cytosol could bypass endosomal antigen delivery and subsequent processing with MHC class II molecules, which occurs when the antigen is delivered alone or in alum. MHC class II molecules primarily induce an antibody response but not or little cell specific-immune response. In particular for the treatment and/or prevention of diseases like, for example, viral infections and hyperproliferative diseases a stimulation of a cell specific immune response is required.

Adjuvants may also promote cytosolic antigen delivery and MHC class I presentation by enabling antigens to cross endosomal membranes into the cytosol after ingestion of antigen-adjuvant complexes by APC (Kovacsovics-Bankowskie M. and Rock K.L (1995) 267: 243-246). Furthermore macrophages and dendritic cells can be stimulated by some adjuvants to secrete immunomodulatory cytokines. Various cytokines induced by adjuvants act on lymphocytes to promote predominantly Th1 or Th2 responses (Audibert F.M. and Lise L.D. (1993) Immunol. Today 14: 281; Grun J.L et al. (1989) Cell Immunol. 121: 134). Consequently, several cytokines including IFN-γ, GM-CSF and interleukin-(IL)-12, are currently under evaluation as vaccine adjuvants.

Another set of adjuvants act through toll-like receptors. Toll-like receptors (TLR) recognize specific patterns of microbial components, especially those from pathogens, and regulate the activation of both innate and adaptive immunity (Takeda et al. (2003) Annu.Rev. Immunol. 21, 335-376). Immature dendritic cells mature in response to these microbial components. As of yet 10 members of the TLR-family have been identified. TLR are expressed by phagocytic cells such as monocytes, macrophages and dendritic cells. TLR activation through ligand binding leads to signal transduction events either in a MyoD88-dependent pathway (NF-κB) or MyoD88-independent pathway (IFR-3).

While vaccines comprising adjuvants have been used very successfully to protect individuals against developing infectious diseases, in particular infectious disease, which the body attacks through a humoral immune response, they have only been employed with limited success in the prevention and treatment of diseases, which can not be attributed to external pathogens and/or which require a cell-mediated immune reaction, like, for example, viral infections or hyperproliferative diseases. In particular hyperproliferative diseases are often caused or accompanied by a variety of alterations of proteins of the diseased cell and therefore, it has been proposed, that these alterations might be used to allow the immune system of the patient to specifically detect and destroy the diseased cells. This type of approach has been called depending on the point at which it is applied protective or therapeutic cancer vaccination. However, for this new treatment or prevention strategies to be successful it is required that the patient mounts a strong and specific immune response against the respective antigens like, for example, against all cells expressing an altered protein.

Even so many antigens, which are specific to certain diseases and which are, thus, suitable to be administered as a therapeutic or protective vaccine are known the specific immune response obtained is often not sufficient to provide the patient with an efficient protection let alone to treat a patient with an already established disease. Currently most adjuvants are administered to the patient together with the antigen in a free form, which means that they are in solution and not attached or incorporated into a vehicle.

It has been described by Mui et al. (2001) J. Pharma. Exp.Therap., 298: 1185 that immune stimulation observed for free CpG ODN is increased, when encapsulated in liposomes. Li et al. (2003) Vaccine, 21: 3319, describe the use of liposomes comprising phosphatidylcholine (PC) and cholesterol (CH) in equimolar amounts with and without 5 mol% phosphatidylethanolamine (PE), which further comprise CpG oligonucleotide and a HER-2/neu derived peptide antigen. This formulation showed an increased immunization, if compared to the use of the free antigen.

Ludewig et al. (2000) Vaccine 19: 23 describe the *in vivo* antigen loading and activation of DC via a liposomal peptide vaccine, comprising CpG ODN and the resulting antiviral and anti-tumor immunity.

Even so prior art liposomal formulations of an antigen and an adjuvant increases the specific immune response, if compared to the immune response elicited by the administration of "free" adjuvant and antigen, there is still a need in the art to further enhance, prolong and improve the immune response, which is elicited by a given antigen. This is the precondition to successfully prevent and cure diseases, in particular those which have not been amenable to vaccination strategies as of yet like, for example, hyperproliferative diseases.

### Detailed Description of the Invention

The present inventors have discovered that the specific immune response against an antigen, which is administered to a patient in a liposome, and the further administration of a first adjuvant, which is either comprised in the first liposome or in a in a second liposome can be markedly enhanced, if a second adjuvant, which is different from the first adjuvant, is coadministered either in free or liposomal form. While some enhancement of the specific immune response has been observed in the past, when two adjuvants were coadministered with an antigen in their free form it was surprising that the enhancement of the adjuvant action observed for two free adjuvants could be even further enhanced, if at least one of the adjuvants and the antigen was comprised in the same or separate liposome(s).

Therefore, the present invention in one aspect relates to a liposome comprising a first adjuvant and at least one second adjuvant, which is different from the first adjuvant, and at least one therapeutic agent.

A second aspect of the invention is a mixture of liposomes comprising a first liposome comprising at least a first adjuvant and at least one therapeutic agent and at least a second liposome comprising at least a second adjuvant, which is different from the first adjuvant.

A third aspect of the invention is a mixture of liposomes comprising a first liposome comprising at least a first adjuvant, a second liposome comprising at least one therapeutic agent and at least a third liposome comprising at least a second adjuvant, which is different from the first adjuvant.

A fourth aspect of the invention is a mixture of liposomes comprising a first liposome comprising at least a first adjuvant, a second liposome comprising at least one therapeutic agent and a liquid medium comprising at least a second adjuvant, which is different from the first adjuvant.

Finally a fifth aspect of the invention is a liposomal composition comprising a liposome comprising a first adjuvant and at least one therapeutic agent and a liquid medium comprising at least a second adjuvant, which is different from the first adjuvant.

In a preferred embodiment the liposome or the mixture of liposomes of the present invention are comprised in a liquid medium. The term "liquid medium" preferably comprises all biocompatible, physiological acceptable liquids and liquid compositions in particular H₂O, aqueous salt solutions, and buffer solutions like, for example, PBS, Ringer solution and the like.

The liposome, the mixture of liposomes or the liposomal composition of the present invention can comprise at least one further component selected from the group consisting of an adjuvant, additive, and auxiliary substance. The term "additive" comprises substances, which stabilize any component of the liposome or of the liquid medium like, for example, antioxidants or radical scavengers or the like. In particular stabilizers are selected from the group consisting of α-tocopherol or carbohydrates, in particular glucose, sorbitol, sucrose, maltose, trehalose, lactose, cellubiose, raffinose, maltotriose, or dextran. The stabilizers can be comprised in the lipid membranes of the liposomes, the interior of the liposomes and/or within the liquid medium surrounding the liposomes.

The term "liposomes" generally refers to uni- or multilamellar (preferably 2, 3, 4, 5, 6, 7, 8, 9, and 10 lamellar) lipid structures enclosing an aqueous interior, depending on the number of lipid membranes formed. Lipids, which are capable of forming a liposomes include all substances having fatty or fat-like properties. Such lipids comprise an extended apolar residue (X) and usually a water soluble, polar, hydrophilic residue (Y), which can be characterized by the basic formula

X-Yₙ

Wherein n equals or is greater than zero. Lipids with n = 0 are termed "apolar lipids", while lipids with n ≥ 1 a referred to as "polar lipids". Preferred lipids, which can make up the lipids in the liposomes of the present invention are selected from the group consisting of glycerides, glycerophospholipides, glycerophosphinolipids, glycerophosphonolipids, sulfolipids, sphingolipids, phospholipids, isoprenolides, steroids, stearines, steroles and carbohydrate containing lipids.

The liposome, at least one liposome comprised in the mixture of liposomes or the liposome comprised in the composition of the present invention comprises in a preferred embodiment steroles, in particular CH, since CH is an abundant constituent of natural membranes and liposomes comprising CH are, therefore, well tolerated and stable. Preferably the liposomes comprise in relation to the total molar lipid composition of the liposome more than about 20 mol% CH.

It has been further recognized by the present inventors, that the presence of the negatively charged lipids, in particular in the presence of CH leads to an increased uptake of the liposomes into cells of the hematopoietic lineage, in particular APCs. Therefore, the liposome, at least one liposome comprised in the mixture of liposomes or the liposome comprised in the composition of the present invention comprises in a further preferred embodiment at least one negatively charged lipid and preferably at least one negatively charged lipid and a sterol, in particular CH. Accordingly it is preferred that the net surface charge of the liposome, at least one liposome comprised in the mixture of liposomes or the liposome comprised in the composition of the present invention is negative, i.e. that the liposome comprises an amount of negatively charged lipids, which exceeds the amount of positively charged lipids in the liposome.

In a preferred embodiment the liposome, at least one liposome comprised in the mixture of liposomes or the liposome comprised in the composition of the present invention the negatively charged lipid is selected from the group consisting of phosphatidylserine (PS), phosphatidylglycerol (PG) and phosphatidic acid (PA).

In an even more preferred embodiment the liposome, at least one liposome comprised in the mixture of liposomes or the liposome comprised in the composition of the present invention comprises CH and at least two components selected from the group consisting of PS, PG and PE. In this case it is even more preferred that the liposome comprises in relation to the total molar lipid composition of the liposome:
a) between 20 mol% and 60 mol% CH; and
b) between 20 mol% and 50 mol% PS;
   between 20 mol% and 50 mol% PG and
   between 20 mol% and 50 mol% PE, respectively.

It has been found that a CH concentration of above 60 mol% in the context of the other lipids is detrimental to the formation of regular lipid bilayer structures and, therefore, a content of 60% CH is the upper limit for the liposome, at least one liposome comprised in the mixture of liposomes or the liposome comprised in the composition of the present invention. On the other hand lowering the cholesterol concentration below 20 mol% appears to decrease the binding and incorporation into hemopoietic cells like, for example DCs. Thus, in a preferred embodiment of the CH comprising liposomes CH is present in relation to the total molar lipid composition of the liposome at a molar ratio of between about 20 to about 60 mol. More preferably CH is present in relation to the total molar lipid composition of the liposome at a molar ratio of about 23 to about 42 mol%, more preferably about 26 to about 39 mol%, even more preferably about 30 to about 36 mol% and most preferably about 32 to about 34 mol%.

In a preferred embodiment PS is present in relation to the total molar lipid composition of the liposome at a molar ratio of about 23 to about 42 mol%, more preferably about 26 to about 39 mol%, even more preferably about 30 to about 36 mol% and most preferably about 32 to about 34 mol%.

In a preferred embodiment PG is present in relation to the total molar lipid composition of the liposome at a molar ratio of about 23 to about 42 mol%, more preferably about 26 to about 39 mol%, even more preferably about 30 to about 36 mol% and most preferably about 32 to about 34 mol%.

In a preferred embodiment PE is present in relation to the total molar lipid composition of the liposome at a molar ratio of about 23 to about 42 mol%, more preferably about 26 to about 39 mol%, even more preferably about 30 to about 36 mol% and most preferably about 32 to about 34 mol%.

In a particular preferred embodiment the liposome, at least one liposome comprised in the mixture of liposomes or the liposome comprised in the composition of the present invention comprises in relation to the total molar lipid composition of each of CH and PS and PG about 23 to about 42 mol%, more preferably about 26 to about 39 mol%, even more preferably about 30 to about 36 mol% and most preferably about 32 to about 34 mol%.

In a particular preferred embodiment the liposome, at least one liposome comprised in the mixture of liposomes or the liposome comprised in the composition of the present invention comprises in relation to the total molar lipid composition of each of CH and PS and PE about 23 to about 42 mol%, more preferably about 26 to about 39 mol%, even more preferably about 30 to about 36 mol% and most preferably about 32 to about 34 mol%.

In a particular preferred embodiment the liposome, at least one liposome comprised in the mixture of liposomes or the liposome comprised in the composition of the present invention comprises in relation to the total molar lipid composition of each of CH and PG and PE about 23 to about 42 mol%, more preferably about 26 to about 39 mol%, even more preferably about 30 to about 36 mol% and most preferably about 32 to about 34 mol%.

The remainder of the lipid, i.e. the amount of lipid which is neither CH, PS and PG; CH, PS and PE; or CH, PG and PE, as the case may be, and which is needed to add up to 100 mol% can be made up of any lipid. Preferred lipids, which can make up the remainder of the lipids in the preferred liposomes of the present invention are selected from the group consisting of glycerides, glycerophospholipides, glycerophosphinolipids, glycerophosphonolipids, sulfolipids, sphingolipids, phospholipids, isoprenolides, steroids, stearines, steroles and carbohydrate containing lipids.

Out of these lipids the remainder of the lipids preferably comprises one or more phospholipids. Preferably the phospholipid is selected from the group consisting of PC and PE. PE can additionally be comprised in those cases in which the preferred liposomes comprise CH, PG and PS.

In a preferred embodiment the liposome, at least one liposome comprised in the mixture of liposomes or the liposome comprised in the composition of the present invention comprises CH, PG and PS in above indicated ranges and preferred ranges further comprises PE in relation to the total molar lipid composition at a concentration of about 1 to about 40 mol%, preferably at about 5 to about 20 mol%, more preferably at about 8 to about 15 mol%.

In a particular preferred embodiment the lipids of the liposome of the present invention essentially consist of CH, PS and PG; CH, PS and PE or CH, PG and PE. In this case CH, PS, PG and/or PE can be present in their preferred and particularly preferred concentration ranges indicated above. Thus, in preferred embodiments the liposomes of the present invention essentially consists in relation to the total molar lipid composition out of CH and PS and PG, wherein each is present in a range of about 23 to about 42 mol%, more preferably about 26 to about 39 mol%, even more preferably about 30 to about 36 mol% and most preferably about 32 to about 34 mol%; out of CH and PS and PE, wherein each is present in a range of about 23 to about 42 mol%, more preferably about 26 to about 39 mol%, even more preferably about 30 to about 36 mol% and most preferably about 32 to about 34 mol%; out of CH and PG and PE, wherein each is present in a range of about 23 to about 42 mol%, more preferably about 26 to about 39 mol%, even more preferably about 30 to about 36 mol% and most preferably about 32 to about 34 mol%.

PS and PG are collective terms for lipids sharing a similar phosphatidylserine and phosphatidylglycerol, respectively, head group. However, many different apolar residues can be attached to these head groups. Thus, PSs and PGs isolated from different natural sources vary substantially in the length, composition and/or chemical structure of the attached apolar residues and naturally occurring PS and PG usually is a mixture of PSs and PGs with different apolar residues. While all PS and PG mixtures or pure isolated or chemically synthesized PS and PG compounds tested so far provide a good immune response when incorporated in the indicated ranges or preferred ranges into a preferred liposome, at least one liposome comprised in the mixture of liposomes or the liposome comprised in the composition of the present invention , it has been observed by the present inventors that certain PS and PG types stimulate a particular strong immune response and, therefore, the PS employed in the liposomes of the present invention is preferably selected from the group consisting of palmitoyloleoylphosphatidylserine, palmitoyllinoeoylphosphatidylserine, palmitoylarachidonoylphosphatidylserine, palmitoyldocosahexaenoylphosphatidylserine, stearoyloleoylphosphatidylserine, stearoyllinoleoylphosphatidylserine, stearoyl-arachidonoylphosphatidylserine, stearoyldocosahexaenoylphosphatidylserine, dicaprylphosphatidylserine, dilauroylphosphatidylserine, dimyristoylphosphatidylserine, diphytanoylphosphatidylserine, diheptadecanoylphosphatidylserine, dioleoylphosphatidylserine, dipalmitoylphosphatidylserine, distearoylphosphatidylserine, dilinoleoylphosphatidylserine dierucoylphosphatidylserine, didocosahexaenoyl-phospahtidylserine, PS from brain, and PS from soy bean; particular preferred is dioleoylphosphatidylserine. The PG employed in the liposome of the present invention is preferably selected from the group consisting of palmitoyloleoylphosphatidylglycerol, palmitoyllinoleoylphosphatidylglycerol, palmitoylarachidonoylphosphatidylglycerol, palmitoyldocosahexaenoylphosphatidylglycerol, stearoyloleoylphosphatidylglycerol, stearoyllinoleoylphosphatidylglycerol, stearoylarachidonoylphosphatidylglycerol, stearoyldocosahexaenoylphosphatidylglycerol, dicaprylphosphatidylglycerol dilauroylphosphatidylglycerol, diheptadecanoylphosphatidylglycerol, diphytanoyl-phosphatidylglycerol, dimyristoylphosphatidylglycerol, dipalmitoylphosphatidylglycerol, dielaidoylphosphatidylglycerol, distearoylphosphatidylglycerol, dioleoylphosphatidylglycerol, dilinoeoylphosphatidylglycerol, diarachidonoylphosphatidylglycerol, docosahexaenoylphosphatidylglycerol, and PG from egg; in particular dioleoylphosphatidylglycerol.

Similar to PS and PG, PE is also a generic term for lipids sharing a phosphatidylethanolamine head group. It has also been observed by the present inventors that certain PEs stimulate a particular strong immune response, if incorporated into the liposome, at least one liposome comprised in the mixture of liposomes or the liposome comprised in the composition of the present invention , therefore, in a preferred embodiment the PE is selected from the group consisting of the PE is selected from the group consisting of palmitoyloleoylphosphatidylethanolamine, palmitoyllinoleoylphosphatidylethanolamine, palmitoylarachidonoylphosphatidylethanolamine, palmitoyldocosahexaenoylphosphatidylethanolamine, stearoyloleoylphosphatidylethanolamine, stearoyllinoleoylphosphatidylethanolamine, stearoylarachidonoylphosphatidylethanolamine, stearoyldocosahexaenoylphosphatidylethanolamine, dilauroylphosphatidylethanolamine, dimyristoylphosphatidylethanolamine, diphytanoylphosphatidylethanolamine, dipalmitoylphosphatidylethanolamine, diheptadecanoylphosphatidylethanolamine, distearoylphosphatidylethanolamine, dielaidoylphosphatidylethanolamine, diarachidonoylphosphatidylethanolamine, docosahexaenoylphosphatidylethanolamine, PE from bacteria, PE from heart, PE from brain, PE from liver, PE from egg, and PE from soybean.

In a preferred embodiment of the liposome, the mixture of liposomes or the liposomal composition of the invention the therapeutic agent is selected from the group consisting of a drug or an antigen.

The term "antigen" as used throughout the specification refers to all substances that elicit an immune response against the antigen in an animal, including a human, upon administration. Such an immune response can be characterized by, for example, a humoral and/or a cell-mediated immune response, which is accompanied by B cell proliferation and antibody secretion, activation of monocytes and/or macrophages as estimated by cytokine secretion (e.g. IL-1, IL-6, TNFα), activation and differentiation of dendritic cells (DC) as estimated by specific expression and/or up-or downregulation of specific surface antigens (e.g. MHC-class II, CD80, CD86, CD83, CD40, DC-LAMP which are upregulated and antigens, e.g. mannose-receptor, DEC-205, DC-SIGN which are downregulated) and by antigen-specific T cells, characterized by their expression of CD4 or CD8 and release of cytokines (e.g. IFNγ) upon activation (restimulation) with the appropriate antigen, in particular the same peptide antigen, used for immune response induction. Drugs in some cases can also elicit an immune response, however, such substances are considered antigens and not drugs, if the detected immune response satisfies the below defined criteria for a tumor antigen. It is preferred that the antigens or fragments thereof are capable of MHC presentation, in particular MHC class I presentation and, therefore, capable to elicit a cell-mediated immune response. In preferred embodiments of the invention the antigen is selected from the group of antigens consisting of a tumor antigen, a viral antigen, a fungal antigen, a bacterial antigen, an autoantigen or an allergen.

The term "tumor antigen" comprises all substances, which elicit an immune response against a tumor. Particular suitable substances are those which are enriched in a tumor cell in comparison to a healthy cell. These substances are preferably present within and/or are accessible on the outside of the tumor cell. If the tumor antigen is only present within a tumor cell, it will still be accessible for the immune system, since the antigen or fragments thereof will be presented by the MHC system at the surface of the cell. In a preferred aspect tumor antigen is almost exclusively present on and/or in the tumor cell and not in a healthy cell of the same cell type.

Suitable tumor antigens can be identified, for example, by analyzing the differential expression of proteins between tumor and healthy cells of the same cell type using a microarray-based approach (Russo et al., Oncogene. 2003, 22:6497-507), by PCR- or microarray-based screening for tumor specific mutated cellular genes (Heller, Annu. Rev. Biomed. Eng. 2002, 4:129-53) or by serological identification of antigens by recombinant expression cloning (SEREX; Tureci et al., Mol Med Today. 1997, 3:342-349). The skilled artisan is aware of a large number of substances which are preferentially or exclusively present on and/or in tumor cell, which include for example, oncogenes like, for example truncated epidermal growth factor, folate binding protein, melanoferrin, carcinoembryonic antigen, prostate-specific membrane antigen, HER2-neu and certain sugar chains like, for example, epithelial mucins.

Not all of the substances that are preferentially or exclusively present in and/or on a tumor cell will elicit a strong immune response, therefore, it is preferred that tumor antigens are selected to be included in the liposomes or in at least one liposome comprised in the mixture of liposomes or comprised in the liposomal composition of the present invention, which elicit a strong immune response, preferentially a MHC class I immune response. Antigens eliciting a strong immune response will induce at least 1%, preferably at least 5%, more preferably at least 10% and most preferably at least 15% IFNγ-producing CD8+ T or CD4+ T cells isolated from mice previously immunized with the antigen, upon challenge with the antigen and/or will induce preferably at least 5%, and most preferably at least 15% of B-cells cells isolated from mice previously immunized with the antigen, upon challenge with the antigen to proliferate. Antigens fulfilling these criterions are candidates for use in therapeutic and/or prophylactic cancer vaccines.

In a particular preferred embodiment the tumor antigen is selected from the group consisting of T-cell-defined cancer-associated antigens belonging to unique gene products of mutated or recombined cellular genes, in particular cyclin-dependent kinases (e.g. CDC2, CDK2, CDK4), p15^{Ink4b}, p53, AFP, β-catenin, caspase 8, p53, p21^{Ras} mutations, Bcr-abl fusion product, MUM-1 MUM-2, MUM-3, ELF2M, HSP70-2M, HST-2, KIAA0205, RAGE, myosin/m, 707-AP, CDC27/m, ETV6/AML, TEL/Aml1, Dekcain, LDLR/FUT, Pml-RARα, TEL/AMLI; Cancer-testis (CT) antigens, in particular NY-ESO-1, members of the MAGE-family (MAGE-A1, MAGE-A2, MAGE-A3, MAGE-A4, MAGE-A6, MAGE-10, MAGE-12), BAGE, DAM-6, DAM-10, members of the GAGE-family (GAGE-1, GAGE-2, GAGE-3, GAGE-4, GAGE-5, GAGE-6, GAGE-7B, GAGE-8), NY-ESO-1, NA-88A, CAG-3, RCC-associated antigen G250; Tumor virus antigens, in particular human papilloma virus (HPV)-derived E6 E7 oncoproteins, Epstein Barr virus EBNA2-6, LMP-1, LMP-2; overexpressed or tissue-specific differentiation antigens, in particular gp77, gp100, MART-1/Melan-A, p53, tyrosinase, tyrosinase-related protein (TRP-1 and TPR-2), PSA, PSM, MC1R; widely expressed antigens, in particular ART4, CAMEL, CEA, CypB, HER2/neu, hTERT, hTRT, iCE, Muc1, Muc2, PRAME RU1, RU2, SART-1, SART-2, SART-3, and WT1; and fragments and derivatives thereof. Particular preferred tumor antigens are antigens derived from the tyrosinase-related protein. The tumor antigen or fragments thereof, selected to be included in a liposome of the present invention preferably stimulates a cell-mediated immune response.

The term "viral antigen" comprises all substances, which elicit an immune response against a virus, in particular against a virally infected cell. It is preferred that the viral antigen elicits a strong immune response as defined above. In preferred embodiments of the present invention the viral antigen is derived from a virus selected from the group consisting of Retroviridae, in particular HIV-1 and HIV-LP; Picornaviridae, in particular polio virus and hepatitis A virus; enterovirus, in particular human coxsackie virus, rhinovirus, echovirus; Calciviridae, in particular strains that cause gastroenteritis; Togaviridae, in particular equine encephalitis virus and rubella virus; Flaviridae, in particular dengue virus, encephalitis virus and yellow fever virus; Coronaviridae, in particular coronavirus; Rhabdoviridae, in particular vesicular stomatitis virus and rabies virus; Filoviridae, in particular Ebola virus or and Marburg virus; Paramyxoviridae, in particular parainfluenza virus, mumps virus, measles virus and respiratory syncytical virus; Orthomyxoviridae, in particular influenza virus; Bungaviridae, in particular Hantaan virus, bunga virus, phlebovirus and Nairo virus; Arena viridae, in particular hemorrhagic fever virus; Reoviridae, in particular reovirus, orbivirus and rotavirus; Birnaviridae; Hepadnaviridae, in particular Hepatitis B virus; Parvovirida, in particular parvovirus; Papovaviridae, in particular papilloma virus, simian virus-40 (SV40) and polyoma virus; Adenoviridae; Herpesviridae, in particular herpes simplex virus (HSV) 1 and 2, varicella zoster virus, cytomegalovirus (CMV), herpes virus; Poxviridae, in particular variola virus, vaccinia virus and pox virus; and Iridoviridae, in particular African swine fever virus; and Hepatitis C. Particularly preferred viral antigens are selected from the group consisting of HPV L6, HPV L7, fragments and derivatives thereof. The viral antigens or fragments thereof, which can be comprised in the liposomes of the present invention, preferably stimulate a cell-mediated immune response.

The term "fungal antigen" comprises all substances, which elicit an immune response against a fungus. It is preferred that the fungal antigen elicits a strong immune response as defined above. In preferred embodiments of the present invention the fungal antigen is derived from a fungus selected from the group consisting of *Cryptococcus* species, in particular *Cryptococcus neoformans, Histoplasma* species, in particular *Histoplasma capsulatum, Coccidioides* species, in particular *Coccidioides immitis, Blastomyces* species, in particular Blastomyces *dermatitidis, Chlamydia* species, in particular *Chlamydia trachomatis,* and *Candida* species, in particular *Candida albicans.* The fungal antigens or fragments thereof, which are preferably comprised in the liposomes of the present invention stimulate a humoral immune response.

The term "bacterial antigen" comprises all substances, which elicit an immune response against a bacterium. It is preferred that the bacterial antigen elicits a strong immune response as defined above. In preferred embodiments of the present invention the bacterial antigen is derived from a bacterium selected from the group consisting of Helicobacter species, in particular *Helicobacter pyloris;* Borelia species, in particular *Borelia burgdorferi;* Legionella species, in particular *Legionella pneumophilia;* Mycobacteria species, in particular *M. tuberculosis, M. avium, M. intracellulare, M. kansasii, M. gordonae;* Staphylococcus species, in particular *Staphylococcus aureus;* Neisseria species, in particular *N. gonorrhoeae, N. meningitidis;* Listeria species, in particular *Listeria monocytogenes;* Streptococcus species, in particular *S. pyogenes, S. agalactiae; S. faecalis; S. bovis, S. pneumoniae;* anaerobic Streptococcus species; pathogenic Campylobacter species; Enterococcus species; Haemophilus species, in particular *Haemophilus influenzae;* Bacillus species, in particular *Bacillus anthracis;* Corynebacterium species, in particular *Corynebacterium diphtheriae;* Erysipelothrix species, in particular *Erysipelothrix rhusiopathiae;* Clostridium species, in particular *C. perfringens, C. tetani;* Enterobacter species, in particular *Enterobacter aerogenes,* Klebsiella species, in particular *Klebsiella pneumoniae,* Pasturella species, in particular *Pasturella multocida,* Bacteroides species; Fusobacterium species, in particular *Fusobacterium nucleatum;* Streptobacillus species, in particular *Streptobacillus moniliformis;* Treponema species, in particular *Treponema pertenue;* Leptospira; pathogenic Escherichia species; and Actinomyces species, in particular *Actinomyces israelli.* The bacterial antigens or fragments thereof, preferably comprised in the liposomes of the present invention stimulate a humoral immune response.

The term "autoimmune antigen" comprises all substances, which elicit an immune response against a substance, e.g. a protein, which is normally present in the body, in particular in a healthy cell, tissue, or organ. Autoimmune antigens can be used for desensitization strategies for the treatment and/or prevention of autoimmune diseases like, for example, type 1 diabetes, conventional organ-specific autoimmune diseases, neurological diseases, rheumatic diseases, psoriasis, connective tissue diseases, autoimmune cytopenias, and other autoimmune diseases. Such conventional organ specific autoimmunity may include thyroiditis (Graves+Hashimoto's), gastritis, adrenalitis (Addison's), ovaritis, primary biliary cirrhosis, myasthenia gravis, gonadal failure, hypoparathyroidism, alopecia, malabsorption syndrome, pernicious anemia, hepatitis, anti-receptor antibody diseases and vitiligo. Such neurological diseases may include schizophrenia, Alzheimer's disease, depression, hypopituitarism, diabetes insipidus, sicca syndrome and multiple sclerosis. Such rheumatic diseases/connective tissue diseases may include rheumatoid arthritis, systemic lupus erythematous (SLE) or Lupus, scleroderma, polymyositis, inflammatory bowel disease, dermatomyositis, ulcerative colitis, Crohn's disease, vasculitis, psoriatic arthritis, exfoliative psoriatic dermatitis, pemphigus vulgaris, Sjogren's syndrome. Other autoimmune related diseases may include autoimmune uvoretinitis, glomerulonephritis, post myocardial infarction cardiotomy syndrome, pulmonary hemosiderosis, amyloidosis, sarcoidosis, aphthous stomatitis, and other immune related diseases, as presented herein and known in the related arts. Autoimmune antigens responsible for the respectively indicated diseases are known in the art and can all without limitation be comprised in a liposome of the present invention.

The term "allergen" refers to a substance, which elicits an immune response against an extraneous substance, which is not a viral, bacterial or fungal antigen. Allergens can be comprised in the liposomes of the present invention for treatment or prevention of allergies by a desensitization strategy. Preferred allergens are selected or derived from the group consisting of pollen, in particular from maple, birch, alder, hazelnut, mugwort, beach mountain cedar, oak, walnut, elm, olive, sycamore, cottonwood, white ash, and white pine; grass, in particular from sweet vernal grass, orchard grass, Bermuda grass, oat grass, rye grass; insects and spiders, in particular mites and bees; food stuff, in particular milk and milk products, nuts, in particular peanuts, hazelnut and almonds; animal hair, in particular hair derived from cat, horse, donkey, sheep, goat, dog, mice, rat, guinea pig, and rabbit. Further allergens, which can be included in the liposomes of the present invention are allergens, which elicit contact hypersensitivity like, for example, nickel and copper.

The liposome, the mixture of liposomes or the liposomal composition of the present can be used for the delivery of a drug in conjunction with two adjuvants and/ if needed additionally with an antigen. In a preferred embodiment the drug is selected from the group consisting of analgesics; antirheumatics; anthelminthics; antiallergics; antianemics; antiarrhythmics; antibiotics; angiogenesis inhibitors; antiinfectives; antidemenics (nootropics); antidiabetics; antidotes; antiemetics; antivertiginosics; antiepileptics; antihemorrhagics; antihypertonics; antihypotonics; anticoagulants; antimycotics; antitussive agents; antiviral agents; beta-receptor and calcium channel antagonists; broncholytic and antiasthmatic agent; chemokines; cytokines, in particular immune modulatory cytokines; mitogens; cytostatics; cytotoxic agents and prodrugs thereof; dermatics; hypnotics and sedatives; immunosuppressants; immunostimulants in particular activators of NF-κB, MAP kinases, STAT proteins and/or protein kinase B/Akt; peptide or protein drugs; in particular hormones and physiological or pharmacological inhibitors of mitogens, chemokines, or cytokines or their respective prodrugs. Of course it is also envisioned that a liposome, at least one liposome comprised in the mixture of liposomes or the liposome comprised in the composition of the present invention comprises more than one drug at once or comprises one or more drugs together with one or more antigens and/or one or more first adjuvants. In a preferred embodiment the drug is selected from the group consisting of chemokines, cytokines, mitogens, cytostatics, cytotoxic agents and prodrugs thereof, immunostimulants, peptide or protein drugs, in particular hormones and physiological or pharmacological inhibitors of mitogens, chemokines, or cytokines or their respective prodrugs.

The liposome, at least one liposome comprised in the mixture or the liposome comprised in the composition of the present invention can comprise any cytostatic or cytotoxic drug, however, from the known cytostatic and cytotoxic drugs the following are particularly preferred: alkylating substances, anti-metabolites, antibiotics, epothilones, nuclear receptor agonists and antagonists, anti-androgenes, anti-estrogens, platinum compounds, hormones and antihormones, interferons and inhibitors of cell cycle-dependent protein kinases (CDKs), inhibitors of cyclooxygenases and/or lipoxygenases, biogeneic fatty acids and fatty acid derivatives, including prostanoids and leukotrienes, inhibitors of protein kinases, inhibitors of protein phosphatases, inhibitors of lipid kinases, platinum coordination complexes, ethyleneimenes, methylmelamines, trazines, vinca alkaloids, pyrimidine analogs, purine analogs, alkylsulfonates, folic acid analogs, anthracendiones, substituted urea, methylhydrazin derivatives, in particular acediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin, or their respective derivatives or analogs thereof. Several of the above indicated drugs are now administered simultaneously for cancer therapy and, consequently, it is also envisioned that more than one cytostatic and/or cytotoxic drug is comprised in a liposome of the present invention.

In preferred embodiments the liposome, at least one liposome comprised in the mixture of liposomes or the liposome comprised in the composition of the present invention comprises the therapeutic agent in an amount such that the ratio of the molar amount of therapeutic agent to the molar amount of total lipids is between 1:100 and 1:10, preferably between 1:80 and 1:15 and more preferably between 1:50 and 1:20.

Liposomes of the present invention can have a diameter between 10 and 1000 nm. They, however, have in a preferred embodiment a diameter of between 50 and 200 nm and more preferably between 100 and 180 nm. The diameter of the liposomes can be affected, for example, by extrusion of the liposomal composition through sieves or meshes with a known pore size. This and further methods of controlling the size of liposomes are well known in the art and are described, for example, in Mayhew et al. (1984) Biochim. Biophys. Acta 775:169-174 or Olson et al. (1979) Biochim. Biophys. Acta 557:9-23.

The term "adjuvant" as used herein refers to substances, which when administered prior, together or after administration of an antigen accelerates, prolong and/or enhances the quality and/or strength of an immune response to the antigen in comparison to the administration of the antigen alone. Preferably the first and the second adjuvants, which can be comprised in the liposome, the mixture of liposomes or the liposome comprised in the liposomal composition of the present invention each individually increase the response to an antigen at least by 20%, preferably at least by 50%, more preferably at least by 100%, more preferably at least by 200% and most preferably by at least a 1000%. The response to an antigen and the increase caused by an adjuvant can be measured by any of a variety of art known methods including the methods outlined above with respect to tumor antigens. Preferably the at least two different adjuvants are selected from the group of adjuvants consisting of unmethylated DNA, in particular unmethylated DNA comprising CpG dinucleotides (CpG motif), in particular CpG ODN with phosphorothioate (PTO) backbone (CpG PTO ODN) or phosphodiester (PO) backbone (CpG PO ODN); bacterial products from the outer membrane of Gram-negative bacteria, in particular monophosphoryl lipid A (MPLA), lipopolysaccharides (LPS), muramyl dipeptides and derivatives thereof; synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, interleukin- (IL-)2, IL-6, IL-7, IL-18, type I and II, interferons, in particular interferon-gamma, TNF-alpha; 25-dihydroxyvitamin D3 (calcitriol); synthetic oligopeptides, in particular MHCII-presented peptides; gel-like precipitates of aluminum hydroxide (alum). Particular preferred adjuvants, which can be comprised in the liposome of the present invention are selected from the group unmethylated DNA, in particular unmethylated DNA comprising CpG dinucleotides (CpG motif), in particular CpG ODN with phosphorothioate (PTO) backbone (CpG PTO ODN) or phosphodiester (PO) backbone (CpG PO ODN), bacterial products from the outer membrane of Gram-negative bacteria, in particular monophosphoryl lipid A (MPLA) and synthetic lipopeptide derivatives, in particular Pam₃Cys.

The liposome, the liposomes comprised in the mixture of liposomes or the liposome comprised in the liposomal composition of the invention can comprise various amounts of the respective adjuvants. Since it is desirable to deliver as much of the first adjuvant as possible together with the therapeutic agent to elicit the maximum adjuvant effect, therefore, adjuvants are usually supplied in excess during formation of the liposome in order to encapsulate as much of the adjuvant together with the therapeutic agent as possible. Depending on the chemical composition and properties of the liposome and the respective adjuvant to be encapsulated and the method used for formation of the liposome, different encapsulation efficiencies will be obtained and, thus, the liposomes of the present invention will comprise different amounts of adjuvants. In preferred embodiments, in which the liposomes comprise CH and at least one negatively charged lipid the liposomes comprises lipophilic adjuvants incorporated at concentrations between 0.1 and 10 mol% or hydrophilic adjuvants encapsulated at a concentration of 1 to 100 µg per µmol lipid.

The present inventors have discovered that liposomes comprising an antigen and an adjuvant can elicit substantially enhanced immune responses against antigens, if coadministered with a second adjuvant, which is different from the first adjuvant. Albeit it is known in the art that an immune response can be stimulated even further when an adjuvant is co-administered with an antigen it was surprisingly found that the combined effect of two adjuvants was stronger, if at least one was comprised in a liposome than the effect of two coadministered "free" adjuvants. The first adjuvant or the second adjuvant, in cases in which the second adjuvant is also comprised within the same or a different liposome, can be comprised "freely" within the interior of the liposome, in particular if the adjuvant is hydrophilic, can be comprised in the membrane of the liposome, in particular, if the adjuvant is lipophilic, or it can be attached to any component making up the liposome like, for example, a lipid component of the liposome, preferably, PE, PS and/or PG, or a protein comprised within the liposome, e.g. can be attached to the antigen.

The liposome, the liposomal mixture or the liposomal composition of the present invention comprise two or more adjuvants of which at least one is incorporated into a liposome. Preferably the two or more adjuvants will act synergistically in the stimulation of the cells of the immune system in particular APCs like, for example, dendritic cells, macrophages and Langerhans cells. A synergistic effect of two adjuvants can in particular be observed, if the adjuvants bind to different receptors or stimulate different molecular pathways, which are involved in the mediation of the adjuvant effect. Thus, it is preferred that each of the first and the second adjuvant stimulates the immune response to the antigen primarily through a different receptor. Albeit some adjuvants like, for example heat shock proteins are capable to stimulate more than one receptor simultaneously. In those cases it is preferred that the second antigen stimulates yet another receptor. Receptors, which stimulate an immune response are known to the skilled artisan and comprise, for example, cytokine receptors, in particular type I cytokine receptors, type II cytokine receptors, TNF receptors; and vitamin D receptor acting as transcription factor; and the Toll-like receptors 1 (TLR1), TLR-2, TLR 3, TLR4, TLR5, TLR-6, TLR7 and TLR9. It is, therefore, preferred that the first and the second adjuvant activate at least two receptors selected from the group consisting of the type I cytokine receptors, type II cytokine receptors, TNF receptors; vitamin D receptor; and the TLR1, TLR-2, TLR 3, TLR4, TLR5, TLR-6, TLR-7 and TLR-9. The skilled artisan knows adjuvants, which activate the respective receptors.

In a preferred embodiment the liposome, the liposomal mixture or the liposomal composition of the present invention comprises a first and the second adjuvant, which primarily stimulate different receptors are selected for:
a) type I cytokine receptors from the group consisting of GM-CSF, IL-2, IL-6 and IL-7;
b) type II cytokine receptors from the group consisting of IFN-α/β and IFN-γ;
c) TNF receptors from the group consisting of TNF-α and CD40 ligand;
d) vitamin D receptor from the group consisting of calcitriol;
e) TLR-1 from the group consisting of tri-acyl lipopeptides from bacteria and mycobacteria and soluble factors from Neisseria meningitides;
f) TLR-2 from the group consisting of lipopeptides, in particular Pam₃Cys, lipoarabinomannan from mycobacteria, peptidoglycan, zymosan and heat shock proteins (HSPs), in particular HSP70, ; lipoteichoic acid from gram-positive bacteria, phenol-soluble modulin from Staphylococcus species., in particular *Staphylococcus epidermidis,* glycoinositolphospholipids from Trypanosoma species, in particular *Trypanosoma cruzi,* glycolipids from Treponema maltophilum, porins from Neisseria, atyptical LPS from Leptospira species, in particular *Leptospira interrogans* and Porphyromonas species, in particular *Porphyromonas gingivalis;*
g) TLR-3 from the group consisting of viral double-stranded RNA and poly dI:dC;
h) TLR-4 from the group consisting of LPS from gram-negative bacteria and its derivatives, in particular monophosphoryl lipid (MPLA), HSPs in particular HSP60 and HSP70, Taxol, fusion proteins of RSV, envelope protein of MMTV, fibronectin and fragments thereof, oligosaccharides of hyaluronic acid, polysaccharide fragments of heparan sulfate and fibrinogen;
i) TLR-5 from the group consisting of bacterial flagellin;
j) TLR-6 from the group consisting of di-acyl lipopeptides from mycoplasma;
k) TLR-7 from the group consisting of imidazoquinoline, loxoribine and bropirimine; and
l) TLR-9 from the group consisting of unmethylated DNA, in particular CpG-DNA; unmethylated CpG oligonucleotides, in particular phosphorothioate CpG-PTO oligonucleotides.

In a further embodiment any of the components making up the membrane of the liposome, of the liposome comprised within the liposomal mixture or of the liposome within the liposomal composition of the present invention can be attached to a further chemical moiety. The term chemical moiety is not particular limited. However, in preferred embodiments the chemical moiety is a targeting moiety as discussed in more detail below or a stabilizing moiety.

The term "attached" as used throughout this description refers to a direct or indirect, covalent or non-covalent bond and connection, respectively, between a chemical moiety in particular a targeting moiety or stabilizing moiety and another component of the liposome, in particular a direct covalent bond. A wide variety of chemical groups which allow attachment as defined above are known in the art including, for example, biotin-streptavidin, amino-reactive groups (e.g. carbodiimides, hydroxylmethylphosphine, imidoester, N-hydroxysuccinimide esters, isothiocyanates, isocyanates), sulfhydryl-reactive groups (e.g. maleimides, haloacetyls, pyridyl disulfides, aziridines) carboxyl-reactive molecules (e.g. carbodiimides, carbodiimidazole, diaoalkanes), hydroxyl-reactive groups (e.g. carbonyldiimidazole, alkyl halogens, isocyanates), and can readily be selected by someone of skill in the art as appropriate.

Stabilizing moieties within the meaning of this invention increase the circulation time of the liposome once they are administered. Particular preferred stabilizing moieties are ganglioside GM1, phosphatidylinositol or PEG, particular preferred PEGs have a molecular mass between about 1,000 and about 10,000 g/mol, more preferably about 5,000 g/mol.

In a preferred embodiment the chemical moieties in particular the stabilizing moieties are attached to only a fraction of the molecules making up the membrane of the liposomes. It is preferred that between about 1 to about 20 mol% of the components of the liposomal membrane carry an attached chemical moiety, more preferably between about 3 and about 10 mol% and even more preferably about 5 mol%.

A preferred liposomal component for attachment of the chemical moiety, in particular for the stabilizing moiety is a lipid component. While different chemical moieties can be attached to different lipid components it is preferred that the chemical moiety(ies) is(are) attached to one or more of the phospholipids comprised within the preferred liposomes of the present invention. In a further preferred embodiment the one or more chemical moiety is attached to PE. In particular, if a stabilizing agent like, for example, PEG is used PE is used for attachment.

In addition to the attachment of stabilizing moieties detergents, proteins and peptides can be incorporated into the liposome for stabilizing the lipid bilayers of the liposomes of the present invention. Detergents which can be used as bilayer stabilizing components include, but are not limited to, Triton X-100, deoxycholate, octylglucoside and lysophosphatidylcholine. Proteins which can be used as bilayer stabilizing components include, but are not limited to, glycophorin and cytochrome oxidase. In preferred embodiments a liposome can comprise between 0.05 and 15 mol% of a stabilizing agent.

It is known that depending on the lipid composition of the liposome some show a preferential binding to certain cell types. For example, the preferred liposomes of the present invention, which comprise CH and a negatively charged lipid, in particular at least two components selected from the group consisting of PS, PG and PE exhibit a preference for binding to cells of the hematopoietic lineage. In particular for vaccination strategies it is desirable that an antigen is even more specifically delivered to cells of the hematopoietic lineage like, for example, to APSs. Other liposomes of the present invention, which do not show a preference for (a) particular cell type(s) can be provided with a means of targeting, which allows targeting of such liposomes primarily to a specific cell type or cell types within the body. Similarly such targeting means can further enhance the cell type specificity of liposomes, which show a cell-type specificity because of their liposomal composition. More specific targeting can aid in decreasing unwanted systemic effects and/or toxicity or it can enhance the immune response by more effectively delivering the antigen and the adjuvant to, for example, APS. Therefore, in a further embodiment of the liposome, the mixture of liposomes or the liposomal composition of the present invention a targeting moiety is attached to at least one of the liposomes. As outlined above with respect to the chemical moiety the targeting moiety can be attached to any component of the liposome. Preferably, the targeting moiety is: a) attached to one of the lipid components of the liposome, b) attached to a membrane protein which can be incorporated into the membrane of the liposomes of the present invention or c) is itself capable of insertion or integration in the lipid layer.

In a preferred embodiment the targeting moiety is selected from the group consisting of a peptide or protein, in particular an antibody or fragment thereof, a single-chain antibody or fragment thereof, a receptor ligand or fragment thereof; an aptamer; and a carbohydrate.

More specifically the targeting moiety can be selected from the group consisting of natural or synthetic receptor-binding peptides and mimetics thereof, mono- or oligosaccharides, receptor ligands or fragments thereof, antibodies or fragments thereof, all of which are directed against DC-specific surface molecules or receptors, in particular CD54 (ICAM-1) and ICAM-2, mannose receptor, CD207 (langerin), ASGPR, CLEC-1, CLEC-2, DCIR, dectin-1, DC-SIGN, DEC-205, BDCA-2, TLR-1, TLR-2, TLR-3, TLR-4, TLR-5, TLR-7, TLR-9, CD40, CD 16/32 (FcyR-III and -II), CD11, CD1a, CD1d, and MHC class II.

In a preferred embodiment the targeting moiety is attached to a spacer. The term "spacer" as used throughout the description refers to a chemical moiety, which serves the purpose of providing better accessibility of the targeting moiety even when it is attached to a component of the liposome, e.g. a lipid, which might otherwise sterically hinder the binding of the targeting moiety to its respective target structure. Spacers within this meaning have a linear extension of at least 0.5 nm preferably the spacer has a linear extension of between 1 and 10 nm and even more preferably between 2 and 5 nm. The spacer is preferably a linear or branched saturated or unsaturated carbohydrate chain. The carbohydrate chain preferably comprises multimeric repeats of a monomeric building block. Depending on the length of the respective monomeric building block between 2 and 10 multimeric repeats of the monomeric building blocks are preferred. In preferred embodiments the spacer is hydrophilic. The spacer can comprise a functional group which allows attachment to the targeting moiety on one terminus and another functional group on the other terminus, which allows attachment of the spacer to a component of the liposome, e.g. a lipid of the present invention.

Preferred spacers are bifunctional molecules, in particular, bifunctional polyethylene or polypropylene glycol derivatives comprising preferably between about 1 and 40 repeat units, oligopeptides comprising natural and/or synthetic amino acids. The oligopeptides preferably comprise between 1 and 40, preferably between 2 and 20 and more preferably between 2 and 10 amino acids. A particular preferred building block of a spacer is 8-amino-3, 6-dioxatanoic acid (doo) and spacers comprising between 1 to 10 repeat units of doo are preferred. Spacers comprising between 2 and 5 doo units are even more preferred and spacers comprising 3 doo units are most preferred. In the context of liposomes it has been discovered by the present inventors that there is an optimal length of the spacer, which is between 2 and 5 nm. On one hand spacers with a length of less than about 0.5 nm will in most cases not provide enough distance from the liposomal surface to which the targeting moiety has been attached to allow efficient interaction, i. e. binding, between the targeting moiety and its respective target like, for example, a tumor cell. On the other hand spacers, which are longer than about 10 nm show an increasing "floppiness", which is also detrimental to the interaction between the targeting moiety and its target. Thus, in a preferred embodiment the spacer has a length of between about 1 and about 10 nm, preferably between about 2.5 and about 5 nm.

In preferred embodiments of liposomes of the present invention the targeting moiety is attached to a lipid, preferably a phospholipid like, for example PE, PG, PC or PS and preferably the lipid, which is used for attachment of a targeting moiety is selected from the group consisting of N-caproylamine-PE, N-dodecanylamine-PE, phophatidylthioethanol, N-[4-(p-maleimidomethyl)cyclohexane-carboxamide-PE (N-MCC-PE), N-[4-(p-maleimidophenyl)butyramide]-PE (N-MPB), N-[3-(2-pyridyldithio)propionate]-PE (N-PDP), N-succinyl-PE, N-glutaryl-PE, N-dodecanyl-PE, N-biotinyl-PE, N-biotinyl-cap-PE, phosphatidyl-(ehtylene glycol), PE-polyethylene glycol (PEG)-carboxylic acid, PE-PEG-maleimide, PE-PEG-PDP, PE-PEG-amine, PE-PEG-biotin, PE-PEG-HNS, dipalmitoylglycerosuccinyl-lysine, alpha-methoxy-omega-(1,2-dioctadecenoyloxy glyceryl) (DO), alpa-methoxy-omega-(1,2-ditetradecenoyloxy glyceryl) (DT).

As outlined above the main components and in many embodiments the only components making up the membrane of the liposome of the present invention are lipids. However, in some aspects of the present invention the membrane of the liposomes can further comprise components, which are capable of insertion/integration into the lipid layer. Examples of such components are proteins with a hydrophilic portion, including one or more membrane spanning domains or GPI-anchors, or other amphipathic molecules such as lipopeptides and glycolipids or molecules conjugated or fused to one or more fatty acid, lipid or other hydrophobic moieties. Such molecules can, for example, provide the liposome with a targeting capacity, i.e. can be a targeting moiety as defined above, or can have an enzymatic function.

It is particularly preferred that the therapeutic or diagnostic compound is comprised in the interior of the liposome or in cases of lipophilic drugs also within or between the lipid bilayers. A variety of methods are available in the prior art to "load" a liposome with a given therapeutic and/or diagnostic agent. In its simplest form the therapeutic or diagnostic agent(s) is(are) admixed with the lipid components during formation of the liposomes. Other passive loading methods include dehydration-rehydration (Kirby & Gregoriadis (1984) Biotechnology 2:979), reverse-phase evaporation (Szoka & Papahadjopoulos (1978) Proc. Natl.Acad. Sci. USA 75:4194), or detergent-depletion (Milsmann et al. (1978) Biochim. Biophys. Acta 512:147-155). However, these techniques often lead to a substantial loss of therapeutic and/or diagnostic agent during loading, which is a particular disadvantage in cases where the therapeutic or diagnostic agent is expensive.

Other methodologies for encapsulating therapeutic and/or diagnostic agents include so called "remote loading" or "active loading" in which due to a gradient, for example, a pH or salt gradient between the exterior and the interior of a preformed liposome the therapeutic or diagnostic agent is transported into the liposome along the gradient (see, for example, Cheung et al. (1998) Biochim. Biophys. Acta 1414:205-216; Cullis et al. (1991) Trends Biotechnol. 9:268-272; Mayer et al. (1986) Chem. Phys. Lipids 40:333-345).

Most active and passive loading procedures require the solubilization of the therapeutic and/or diagnostic compound in a solvent. For some compounds in particular hydrophobic compounds or compounds of higher molecular weight, like, for example, peptides or proteins the solubilization in an aqueous solvent can proof difficult and this can make loading inefficient and, thus, in particular with expensive compounds uneconomical. Thus, rather than using an aqueous solvent in those cases organic solvents have been used in the prior art. However, the administration of liposomes or liposomal compositions comprising organic solvents is often not feasible since they pose biocompatibility problems and, therefore, the organic solvents have to be removed prior to administration. The present inventors, however, have now discovered that the liposomes of the present invention can efficiently be produced with a method comprising the steps of:
a) forming a suspension of at least one lipid, one or more therapeutic agent and optionally a first and/or a second adjuvant in a liquid medium, and
b) homogenizing the suspension.

In a preferred embodiment the lipid or lipids, the therapeutic agent and/or at least one of the adjuvants are essentially not soluble in the liquid medium. Preferably the therapeutic and agent is essentially not soluble. Preferred liquid mediums are H₂O, aqueous salt solutions and/or buffer solutions. Preferably the lipid or lipids are the preferred lipids and lipid compositions indicated above and the lipids and therapeutic agents are employed in above indicated ranges and preferred ranges.

Further passive and active loading techniques are well known in the art and can all without limitation be employed by the skilled artisan to produce the liposome, at least one liposome of the mixture of liposomes or the liposome comprised in the liposomal composition of the present invention. The most efficient method of loading for any given therapeutic agent and/or adjuvant can be determined by routine experimentations by well established procedures. Variables which are typically adjusted are pH, temperature, salt type and concentration, type of buffer, solvent etc.

In a preferred embodiment the therapeutic agent and/or at least the first adjuvant is (are) loaded by remote loading into the liposomes, since this method offers a very low loss of the substance to be loaded. In a preferred embodiment a pH gradient is used for loading. Depending on the substance to be loaded the interior of the liposome will typically be acidified with respect to its exterior. Preferably the interior will have a pH between 1 and 6 prior to loading with the therapeutic and/or diagnostic agent.

Therefore, another aspect of the present invention is a liposome produced by one of the above methods, in particular the method of forming a suspension of lipids comprising CH, and at least two components selected from the group consisting of PS, PG, and PE one or more therapeutic and/or diagnostic agent and a liquid medium and homogenizing the suspension.

It was shown that the liposome, the mixture of liposomes or the liposomal composition of the present invention is more suitable for vaccination strategies aiming at the treatment or prevention of diseases than the prior art vaccine formulations and, therefore, any disease, which is known or suspected to be treatable or preventable by a vaccination strategy can more successfully be treated or prevented with the liposome, the mixture of liposomes or the liposomal compositions of the present invention. Thus, another aspect of this invention is the use of the liposomes, the mixtures of liposomes or the liposomal compositions for the production of a medicament for the prevention or therapy of proliferative diseases, infectious diseases, vascular diseases, rheumatoid diseases, inflammatory diseases, immune diseases, in particular autoimmune diseases and allergies. Thus produced medicaments can be used to treat or prevent diseases in animals, including human beings.

The liposomes, the mixture of liposomes or the liposomal composition can be administered through a variety of ways including intra-muscular, intravenous, intranasal, intraperitoneal, intradermal, or subcutaneous and intranodal application. The compounds can also be injected directly into the disease site. The liposomes are administered in amounts and intervals, which are commonly used for other vaccination/immunization strategies or in the case of the delivery of drugs at a dose, which is commonly used for the free drug.

In the experiments performed by the present inventors it was shown that the liposome, the mixture of liposomes or the liposomal composition of the present invention has a superior efficacy in the treatment and/or prevention of tumors and, therefore, in a preferred embodiment the proliferative disease to be treated or prevented is selected from the group consisting of carcinomas of the gastrointestinal or colorectal tract, liver, pancreas, kidney, bladder, prostate, endometrium, ovary, testes, melanoma, dysplastic oral mucosa, invasive oral cancers, small cell and non-small cell lung carcinomas, hormone-dependent breast cancers, independent breast cancers, transitional and squamous cell cancers, neurological malignancies including neuroblastoma, gliomas, astrocytomas, osteosarcomas, soft tissue sarcomas, hemangioamas, endocrinological tumors, hematologic neoplasias including leukemias, lymphomas, and other myeloproliferative and lymphoproliferative diseases, carcinomas in situ, hyperplastic lesions, adenomas, fibromas, histiocytosis, chronic inflammatory proliferative diseases, vascular proliferative diseases and virus-induced proliferative diseases.

In a preferred use of the liposomes, the mixtures of liposomes or the liposomal compositions of the invention one or more adjuvants and or cytokines are administered prior, simultaneously or after administration of the liposome or liposomal composition. The term adjuvant is used here as previously defined. Preferred adjuvants are selected from the group of adjuvants consisting of unmethylated DNA comprising CpG dinucleotides (CpG motif), in particular CpG ODN with phosphorothioate (PTO) backbone (CpG PTO ODN) or phosphodiester (PO) backbone (CpG PO ODN); gel-like precipitates of aluminum hydroxide (alum); bacterial products from the outer membrane of Gram-negative bacteria, in particular monophosphoryl lipid A (MPLA), lipopolysaccharides (LPS), muramyl dipeptides and derivatives thereof; synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, interleukin-(IL-)2, IL-6, IL-7, IL-18, type I and II, interferons, in particular interferon-gamma, TNF-alpha; oil in water emulsions, in particular MF59 consisting of squalene; Tween 80 and Span 85 (sorbitan-trioleate) and QS-21, a more highly purified derivative of Quil A, non-ionic block polymers, in particular Poloxamer 401, saponins and derivatives thereof, in particular the immunostimulatory fragments from saponins; polyphosphazene; N-(2-Deoxy-2-L-leucylamino-β-D-glucopyranosyl)-N-octadecyldodecanoylamide hydroacetate (BAY R1005), 25-dihydroxyvitamin D3 (calcitriol); DHEA; murametide [MDP(Gln)-OMe]; murapalmitine; polymers of lactic and/or glycolic acid; polymethyl methacrylate; sorbitan trioleate; squalane; stearyl tyrosine; squalene; theramide, synthetic oligopeptides, in particular MHCII-presented peptides. Particular preferred adjuvants, which can be administered prior, during or after administration of the liposomes, the mixtures of liposomes or the liposomal compositions of the present invention are cytokines with adjuvant activity, in particular GM-CSF, IL-2, IL-6, IL-7, IL-18, type I and II, interferons, in particular interferon-gamma, or TNF-alpha.

The liposomes comprising two adjuvants of the mixture of liposomes, wherein each comprises at least one different adjuvant are stable structures, which can, for example, be filtered after production to remove surrounding drug solutions or buffers. The "pure" liposomes with or without therapeutic agent and/or adjuvant(s) can be used, however, due to its stability it is also possible to remove essentially all liquid from the liposome and the mixture of liposomes, respectively, to facilitate easy storage in a dried state. Therefore, the liposome or the mixture of liposomes of the present invention can be supplied in dried form, preferably in a freeze dried form. These liposomes can be readily rehydrated upon addition of, for example, water, salt solution and/or buffer at the time and point of use.

The following examples are included to demonstrate preferred embodiments of the invention. It should be appreciated by those of skill in the art that the techniques disclosed in the examples that follow represent techniques discovered by the inventors to function well in the practice of the invention, and thus can be considered preferred modes for its practice. However, those of skill in the art should, in light of the present disclosure, appreciate that many changes can be made in the specific embodiments that are disclosed without departing from the spirit and scope of the invention as set out in the appended claims. All references cited are incorporated herein by reference.

### Brief Description of the Figures and Drawings

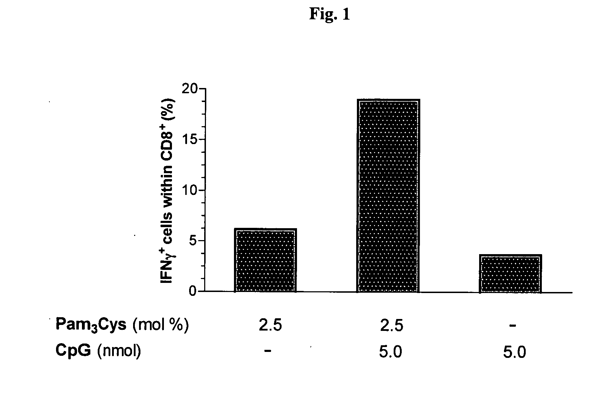

**Fig. 1: Synergistic adjuvant effect between CpG-PTO and Pam**_{**3**}**Cys.** Mice were immunized twice with Pam₃Cys-AVE3/TRP-2; or Pam₃Cys-AVE3/TRP-2 plus 5 nmol CpG in saline; or AVE3/TRP-2 plus 5 nmol CpG in saline applying 10 µg TRP-2 and 21 µg Pam₃Cys per animal. IFNγ⁺ producing cells of the CD8⁺ fraction were determined by cytokine secretion assay 16 hrs after re-stimulation in vitro with 5 µM TRP-2 peptide. As negative control unstimulated cells or cells restimulated with 5 µM OVA peptide were included. In all cases background levels were between 0.5 to 1.5% IFN⁺ producing CD8⁺ cells (not shown).

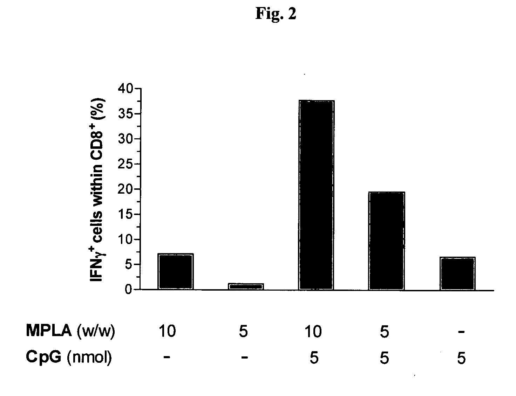

**Fig. 2: Synergistic adjuvant effect between CpG-PTO and MPLA.** Mice were immunized twice with MPLA-AVE3/TRP-2 containing 5 or 10% (w/w) MPLA, or MPLA-AVE3/TRP-2 plus 5 nmol CpG in saline, or AVE3/TRP-2 plus 5 nmol CpG in saline applying 10 µg TRP-2 and 33 or 66 µg MPLA per animal. IFNγ⁺ producing cells of the CD8⁺ fraction were determined by cytokine secretion assay 16 hrs after re-stimulation in vitro with 5 µM TRP-2 peptide. As negative control unstimulated cells or cells restimulated with 5 µM OVA peptide were included. In all cases background levels were between 0.5 to 1.5% IFN⁺ producing CD8⁺ cells (not shown).

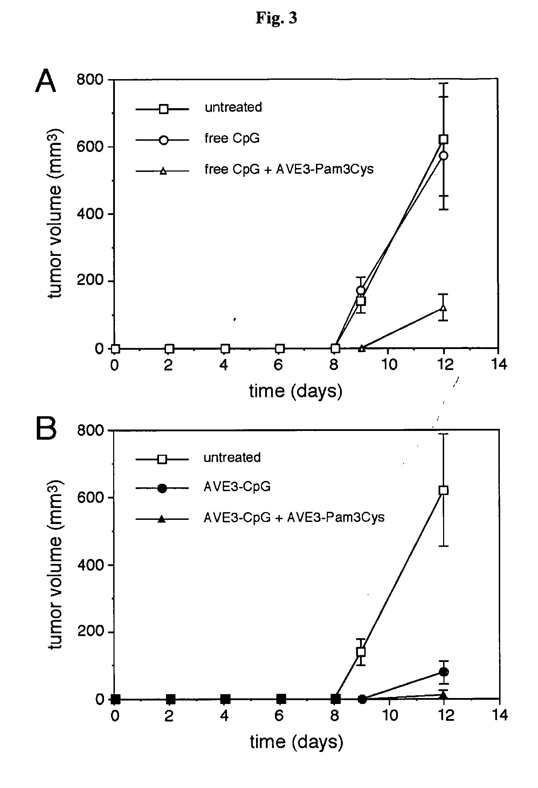

**Fig. 3:** Low doses of liposomal vaccination prevent tumor growth. Mice (n = 4) were immunized twice with 10 µg TRP-2 either in AVE3 or AVE3-Pam₃Cys in the presence of 5 nmol free CpG-PTO (A), or the presence of 1.3 nmol AVE3-encapsulated CpG-PTO (B). Mice were then inoculated subcutaneously with 2 x 10⁵ B16 melanoma cells at day 0.

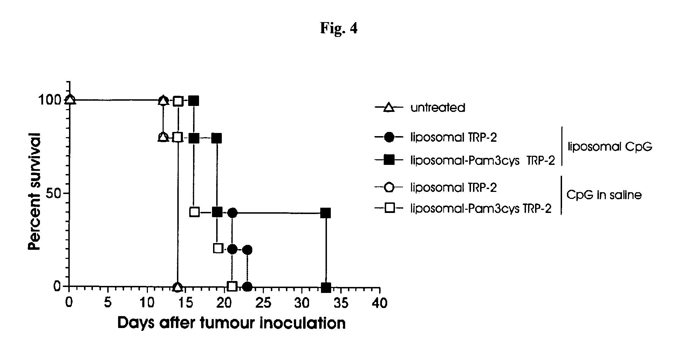

**Fig. 4:** Low doses of liposomal vaccination prolong survival. Mice (n = 4 per group) were immunized twice with AVE3/Pam₃Cys/TRP-2 (10 µg TRP-2 per animal) plus 1.3 nmol liposomal CpG-PTO, AVE3/Pam₃Cys/TRP-2 (10 µg TRP-2 per animal) plus 5 nmol CpG-PTO in saline, liposomal TRP-2 (AVE3/TRP-2) plus 1.3 nmol liposomal CpG-PTO (AVE3/CpG), liposomal TRP-2 (AVE3/TRP-2) plus 5 nmol CpG-PTO in saline or left untreated. Mice were subcutaneously inoculated with 2 x 10⁵ B16 melanoma cells at day 0.

### Examples

### Example 1: Encapsulation of antigenic peptides into AVE3

The antigenic peptide TRP-2 (SVYDFFVWL) was encapsulated into AVE3 (cholesterol, DLPE, DOPS at a molar ratio of 1:1:1). All lipids were purchased from Avanti Polar Lipids (USA) and Calbiochem (USA) and were used without further purification. The lipids were mixed with 29.4 mg TRP-2 at a molar ratio of 1:20 and filled into a 50 ml Duran glass bottle. 50 g Hepes buffer (10 mmol/l, pH 7.4) was added and the mixture was stirred with an Ultra-Turrax T8 dispersing instrument (IKA Werke, Staufen, Germany) for 30 minutes at approx. 25,000 rpm in a 55°C water bath. The Ultra-Turrax was equipped with a S8N-8G dispersing element. The bottle was vortexed several times to avoid precipitations in the comers of the bottle. After obtaining a homogeneous suspension, the evaporated water was replaced and the preparation was transferred in an Emulsiflex C-5 Homogenizer (Avestin Inc., Ottawa, Canada). The homogenization was performed for 30 minutes between 50,000 and 150,000 kPa in front of the homogenization nozzle and a homogenisator pressure of 300 kPa. At the end of this step the filter unit was inserted and the liposomal dispersion was filtered for 5 minutes through polycarbonate membranes with 100 nm pores before being sterile-filtered. After a one-day storage, the particle size was measured by photon correlation spectroscopy and the amount of TRP-2 in the liposomes by HPLC analysis. The size of liposomes was between 130-180 nm. Encapsulation efficiency was in the range of 230-300 µg/ml (39-51%).

### Example 2: Encapsulation of oligonucleotides into AVE3

The lipids (see example 1 for lipid composition of AVE3) dissolved in chloroform or chloroform:methanol (1:1) were dried in a rotary evaporator at 30 mbar and 34°C for 15 minutes. The resulting lipid film was further dried in a vacuum chamber at 10 mbar. The dried film was then hydrated in a rotary evaporator with a few glass beads in 1 ml isotonic Hepes buffer (10 mmol/l; pH 7.4) containing 10 mg/ml oligonucleotides (CpG-1826, 5'-TCCATGACGTTCCTGACGTT-3' as phosphorothioate (PTO) and phosphodiester (PDO)). The dispersion was then extruded 21 times through polycarbonate membranes having pores with a size of 50 nm. In order to remove the free oligonucleotides, the liposomal suspension was subjected to size exclusion chromatography on a sepharose CL-4B column (Pharmacia, Sweden). The collected liposome-fractions were then concentrated by ultrafiltration using Vivaspin concentrators with a cutoff of 30,000 MW (Vivascience, Germany). Finally the vesicles were filtered through 200 nm sterile filter membranes. The hydrodynamic diameter was measured using a Zetasizer 3000 HS (Malvern, Herrenberg, Germany). Encapsulated oligonucleotides were determined by ion-exchange HPLC. The size of liposomes was between 100 to 125 nm. Encapsulation efficiency was in the range of 4 to 7%.

### Example 3: Encapsulation of Pam₃Cys and antigenic peptide into AVE3

All lipids were purchased from Avanti Polar Lipids (USA) and Calbiochem (USA) and Pam₃Cys was purchased from emc (Germany) and used without further purification. Lipids (97.5 mol%, molar ratio of CH:DLPE:DOPS was 1:1:1) as well as the Pam₃Cys (2.5 mol%) dissolved in chloroform or chloroform:methanol (1:1) and the antigenic peptide dissolved in DMSO were dried in a rotary evaporator at 10 mbar and 34°C for 60 minutes. The resulting lipid film was dissolved in chloroform and dried again as described above. This step was repeated until a smooth and homogeneous film was obtained, followed by the removal of residual solvent in a vacuum chamber at 10 mbar. The dried film was then hydrated in a rotary evaporator with a few glass beads in 1 ml isotonic Hepes buffer (10 mmol/l; pH 7.4). The obtained dispersion was extruded 21 times through polycarbonate membranes having pores with a size of 100 nm. At the end of this procedure the formulation was filter sterilized.

### Example 4: Encapsulation of monophosphoryl-lipid A (MPLA) and antigenic peptide into AVE3

All lipids and MPLA were purchased from Avanti Polar Lipids (USA) and used without further purification. Lipids (molar ratio of CH:DLPE:DOPS was 1:1:1) as well as MPLA (5 or 10 % (w/w) in relation to the total lipids) were dissolved in chloroform or chloroform:methanol (1:1) and the antigenic peptide was dissolved in DMSO. The solution was dried in a rotary evaporator at 10 mbar and 34°C for 60 minutes. The resulting lipid film was resolved in chloroform and dried again as described before. This step was repeated till a smooth and homogeneous film was obtained, followed by the removal of residual solvent in a vacuum chamber at 10 mbar. The dried film was then hydrated in a rotary evaporator with a few glass beads in 1 ml isotonic Hepes buffer (10 mmol/l; pH 7.4). The obtained dispersion was extruded 21 times through polycarbonate membranes having pores with a size of 100 nm. At the end of this procedure the formulation was filter sterilized.

### Example 5: Synergistic adjuvant effect of CpG-PTO and Pam₃Cys

Mice were immunized twice at day 0 and day 10 with (i) Pam₃Cys-AVE3/TRP-2 (2.5 mol% Pam₃Cys), or (ii) Pam₃Cys-AVE3/TRP-2 (2.5 mol% Pam₃Cys) plus 5 nmol CpG in saline, (iii) or AVE3/TRP-2 plus 5 nmol CpG in saline applying 10 µg TRP-2 and 21 µg Pam₃Cys per animal. Six days after the second immunization mice were sacrificed, the draining lymph nodes were removed and cells from the lymph nodes were cultured for 6-7 days in the presence of IL-2. Cells were then stimulated with the antigenic peptide (TRP-2) or an irrelevant peptide (OVA peptide; SIINFEKL). After 16 hours CD8-positive cells were analyzed in a cytokine secretion assay for the production of IFNγ. In all experiments dead cells were excluded by propidium iodide staining.

While approximately 6% of the CD8⁺ cell secreted IFNγ in the presence of Pam₃Cys-AVE3/TRP-2 alone, this value increased to over 18% when also CpG was present. CpG alone resulted in approximately 4% secreting cells (Fig. 1). This finding clearly demonstrates that Pam₃Cys-AVE3 together with CpG ODNs have a synergistic effect on the induction of specific IFNγ secreting CD8⁺ cells resulting in approximately twice as many IFNγ secreting CD8⁺ cells as expected from an additive action of Pam₃Cys-AVE3 and CpG ODNs.

### Example 6: Synergistic adjuvant effect of CpG-PTO and monophosphoryl-lipid A (MPLA)

Mice were immunized twice at day 0 and day 10 with (i) MPLA-AVE3/TRP-2 (10% (w/w) MPLA), or (ii) MPLA-AVE3/TRP-2 (5% (w/w) MPLA), or (iii) MPLA-AVE3/TRP-2 (10 (w/w) MPLA) plus 5 nmol CpG-PTO in saline, or (iv) MPLA-AVE3/TRP-2 (5% (w/w) MPLA) plus 5 nmol CpG-PTO in saline, (v) or AVE3/TRP-2 plus 5 nmol CpG-PTO in saline applying 10 µg TRP-2 and 33 or 66 µg MPLA per animal. One week after the second immunization mice were sacrificed, the draining lymph nodes were removed and cells from the lymph nodes were cultured for 6-7 days in the presence of IL-2. Cells were then stimulated with the antigenic peptide (TRP-2) or an irrelevant peptide (OVA peptide; SIINFEKL). After 16 hours CD8-positive cells were analyzed by cytokine secretion assay for the production of IFNγ. In all experiments dead cells were excluded by propidium iodide staining.

Applying MPLA-AVE3/TRP-2 at 5 or 10% (w/w) MPLA (33 or 66 µg MPLA per animal) resulted in approximately 1 or 7% IFNγ secreting CD8⁺ cells, respectively. The addition of CpG ODNs increased the numbers to approximately 19 and 38%, respectively. CpG ODNs alone resulted in approximately 7% INFγ secreting CD8⁺ cells (Fig. 2). Thus, as observed for Pam₃Cys and CpG ODNs, a strong synergistic effect is observed between encapsulated MPLA and CpG ODNs with a 2- to 3-fold increase in the induction of INFγ secreting CD8⁺ cells.

### Example 7: Antitumor effects with Pam₃Cys and CpG-PTO ODNs as adjuvants

Antitumor effects were analyzed in a subcutaneous tumor model of B16.F1 mouse melanoma cells using a prophylactic setting. Mice (C57BL/6) were immunized twice into the hind footpads at a weekly interval. One week after the last immunization mice were inoculated subcutaneous with 2 x 10⁵ B16 tumor cells in a total volume of 200 µl HBSS. The tumor growth after immunization with low doses TRP-2 antigen (10 µg per animal) encapsulated in AVE3, with or without 2.5 mol% Pam₃Cys as liposomal adjuvant, combined with low doses CpG-PTO ODNs (1.3 nmol) in saline or encapsulated into AVE3 was compared. The tumor mass was strongly reduced when mice were immunized with TRP-2 encapsulated in AVE3, with or without Pam₃Cys plus encapsulated CpG-PTO 17 days after B16 inoculation, demonstrating that the encapsulation of the CpG-PTO is necessary to achieve a partial tumor rejection (Fig. 3). In addition, the application of two encapsulated adjuvants (Pam₃Cys and CpG-PTO ODNs) further improved antitumor effects, which is in accordance with the synergistic effects observed ex vivo (example 5). The survival time after B16 melanoma cell transfer (Fig. 4) was also examined. No significant increase of the survival rate could be achieved with AVE3/TRP-2 plus CpG-PTO in saline (mean survival time: untreated, 14 days; AVE3/TRP-2 plus CpG-PTO, 14 days). When mice were immunized with AVE3/Pam₃Cys/TRP-2 plus CpG-PTO in saline the mean survival time significantly increased to 16 days (n = 5; p < 0.0145 AVE3/Pam₃Cys/TRP-2 plus CpG-PTO versus untreated). When mice were immunized with AVE3/TRP-2, with or without Pam₃Cys, plus liposomal CpG-PTO, the mean survival time significantly increased to 19 days (n = 5; p < 0.0035 AVE3/TRP-2 plus AVE3/CpG-PTO versus untreated; n = 5; p < 0.0145 AVE3/Pam₃Cys/TRP-2 plus AVE3/CpG-PTO versus untreated). In addition, these data showed that incorporation of Pam₃Cys into antigen-carrying AVE3 only significantly increases the survival time when the vaccine setting includes unencapsulated CpG-PTO (n = 5; AVE3/TRP-2 plus CpG-PTO versus AVE3/Pam₃Cys/TRP-2 plus CpG-PTO; p < 0.0145).

## Claims

1. A liposome comprising a first adjuvant and at least one second adjuvant, which is different from the first adjuvant, and at least one therapeutic agent.

2. A mixture of liposomes comprising a first liposome comprising at least a first adjuvant and at least one therapeutic agent and at least a second liposome comprising at least a second adjuvant, which is different from the first adjuvant.

3. A mixture of liposomes comprising a first liposome comprising at least a first adjuvant, a second liposome comprising at least one therapeutic agent and at least a third liposome comprising at least a second adjuvant, which is different from the first adjuvant.

4. A mixture of liposomes comprising a first liposome comprising at least a first adjuvant, a second liposome comprising at least one therapeutic agent and a liquid medium comprising at least a second adjuvant, which is different from the first adjuvant.

5. A liposomal composition comprising a liposome comprising a first adjuvant and at least one therapeutic agent and a liquid medium comprising at least a second adjuvant, which is different from the first adjuvant.

6. The liposome or the mixture of liposomes of one of claims 1 to 3, wherein the liposome(s) is(are) comprised in a liquid medium.

7. The liposome, the mixture of liposomes or the liposomal composition of one of claims 4 to 6, wherein the liquid medium is selected from the group consisting of H₂O, aqueous salt solution, and buffer solution.

8. The liposome, the mixture of liposomes or the liposomal composition of any of claims 1 to 7, comprising at least one further component selected from the group consisting of an adjuvant, an additive, and an auxiliary substance.

9. The liposome, the mixture of liposomes or the liposomal composition of any of claims 1 to 8, wherein the lipids of the liposome comprise cholesterol and at least one negatively charged lipid.

10. The liposome, the mixture of liposomes or the liposomal composition of claim 9, wherein the negatively charged lipid comprised in the liposome is selected from the group consisting of phosphatidylserine (PS), phosphatidylglycerol (PG) and phosphatidic acid (PA).

11. The liposome, the mixture of liposomes or the liposomal composition of any of claims 1 to 10, wherein the liposome comprises cholesterol and at least two components selected from the group consisting of PS, PG and PE.

12. The liposome, the mixture of liposomes or the liposomal composition of claim 11, wherein the liposome, comprises in relation to the total molar lipid composition of the liposome:
a) between 20 mol% and 60 mol% CH; and
b) between 20 mol% and 50 mol% PS;
between 20 mol% and 50 mol% PG and
between 20 mol% and 50 mol% PE, respectively.

13. The liposome, the mixture of liposomes or the liposomal composition of any of claims 9 to 12, wherein between one and three components selected from the group consisting of CH, PS, PG and PE is (are) present in relation to the total molar lipid composition of the liposome at a molar ratio of between 30 mol% and 36 mol%.

14. The liposome, the mixture of liposomes or the liposomal composition of any of claims 9 to 13, wherein the remaining lipid of the liposome is selected from the group consisting of glycerides, glycerophospholipides, glycerophosphinolipids, glycerophosphonolipids, sulfolipids, sphingolipids, phospholipids, isoprenolides, steroids, stearines, steroles and carbohydrate containing lipids.

15. The liposome, the mixture of liposomes or the liposomal composition of claim 14, wherein said remaining phospholipid is phosphatidylcholine (PC) or PE.

16. The liposome, the mixture of liposomes or the liposomal composition of one of claims 11 to 15, wherein the lipids of the liposome essentially consist of CH, PS, and PG; CH, PS and PE; CH, PG, and PE; or CH, PG, PS, and PE.

17. The liposome, the mixture of liposomes or the liposomal composition of one of claims 1 to 16, wherein the therapeutic agent is selected from the group consisting of a drug and an antigen.

18. The liposome, the mixture of liposomes or the liposomal composition of claim 17, wherein the antigen is selected from the group of antigens consisting of a tumor antigen, a viral antigen, a fungal antigen, a bacterial antigen, an autoimmune antigen or an allergen.

19. The liposome, the mixture of liposomes or the liposomal composition of claim 18, wherein the tumor antigen is selected from the group consisting of T-cell-defined cancer-associated antigens belonging to unique gene products of mutated or recombined cellular genes, in particular cyclin-dependent kinase 4 (CDK4), p15^{Ink4b}, p53, AFP, β-catenin, caspase 8, p53, p21^{Ras} mutations, Bcr-abl fusion product, MUM-1 MUM-2, MUM-3, ELF2M, HSP70-2M, HST-2, KIAA0205, RAGE, myosin/m, 707-AP, CDC27/m, ETV6/AML, TEL/Aml1, Dekcain, LDLR/FUT, Pml-RARα, TEL/AMLI; Cancer-testis (CT) antigens, in particular NY-ESO-1, members of the MAGE-family (MAGE-A1, MAGE-A2, MAGE-A3, MAGE-A4, MAGE-A6 MAGE-10, MAGE-12), BAGE, DAM-6, DAM-10, members of the GAGE-family (GAGE-1, GAGE-2, GAGE-3, GAGE-4, GAGE-5, GAGE-6, GAGE-7B, GAGE-8), NA-88A, CAG-3, RCC-associated antigen G250; Tumor virus antigens, in particular human papilloma virus (HPV)-derived E6 E7 oncoproteins, Epstein Barr virus EBNA2-6, LMP-1, LMP-2; overexpressed or tissue-specific differentiation antigens, in particular gp77, gp100, MART-1/Melan-A, p53, tyrosinase, tyrosinase-related protein (TRP-1 and TPR-2), PSA, PSM, MC1R; widely expressed antigens, in particular ART4, CAMEL, CEA, CypB, HER2/neu, hTERT, hTRT, iCE, Muc1, Muc2, PRAME RU1, RU2, SART-1, SART-2, SART-3, and WT1; and fragments and derivatives thereof.

20. The liposome, the mixture of liposomes or the liposomal composition of claim 18, wherein the viral antigen is derived from a virus selected from the group of virus consisting of Retroviridae, in particular HIV-1 and HIV-LP; Picomaviridae, in particular polio virus and hepatitis A virus; enterovirus, in particular human coxsackie virus, rhinovirus, echovirus; Calciviridae, in particular strains that cause gastroenteritis; Togaviridae, in particular equine encephalitis virus and rubella virus; Flaviridae, in particular dengue virus, encephalitis virus and yellow fever virus; Coronaviridae, in particular coronavirus; Rhabdoviridae, in particular vesicular stomatitis virus and rabies virus; Filoviridae, in particular Ebola virus or and Marburg virus; Paramyxoviridae, in particular parainfluenza virus, mumps virus, measles virus and respiratory syncytical virus; Orthomyxoviridae, in particular influenza virus; Bungaviridae, in particular Hantaan virus, bunga virus, phlebovirus and Nairo virus; Arena viridae, in particular hemorrhagic fever virus; Reoviridae, in particular reovirus, orbivirus and rotavirus; Birnaviridae; Hepadnaviridae, in particular Hepatitis B virus; Parvovirida, in particular parvovirus; Papovaviridae, in particular papilloma virus, simian virus-40 (SV40) and polyoma virus; Adenoviridae; Herpesviridae, in particular herpes simplex virus (HSV) 1 and 2, varicella zoster virus, cytomegalovirus (CMV), herpes virus; Poxviridae, in particular variola virus, vaccinia virus and pox virus; and Iridoviridae, in particular African swine fever virus; and Hepatitis C.

21. The liposome, the mixture of liposomes or the liposomal composition of claim 18, wherein the fungal antigen is derived from a fungus selected from the group consisting of *Cryptococcus* species, in particular *Cryptococcus neoformans, Histoplasma* species, in particular *Histoplasma capsulatum, Coccidioides* species, in particular *Coccidioides immitis, Blastomyces* species, in particular Blastomyces *dermatitidis, Chlamydia* species, in particular *Chlamydia trachomatis,* and *Candida* species, in particular *Candida albicans.*

22. The liposome, the mixture of liposomes or the liposomal composition of claim 18, wherein the bacterial antigen is derived from a bacterium selected from the group consisting of Helicobacter species, in particular *Helicobacter pyloris;* Borelia species, in particular *Borelia burgdorferi;* Legionella species, in particular *Legionella pneumophilia;* Mycobacteria species, in particular *M. tuberculosis, M. avium, M. intracellulare, M. kansasii, M. gordonae;* Staphylococcus species, in particular *Staphylococcus aureus;* Neisseria species, in particular *N. gonorrhoeae, N. meningitidis;* Listeria species, in particular *Listeria monocytogenes;* Streptococcus species, in particular *S. pyogenes, S.* *agalactiae; S. faecalis; S. bovis, S. pneumoniae;* anaerobic Streptococcus species; pathogenic Campylobacter species; Enterococcus species; Haemophilus species, in particular *Haemophilus influenzae;* Bacillus species, in particular *Bacillus anthracis;* Corynebacterium species, in particular *Corynebacterium diphtheriae;* Erysipelothrix species, in particular *Erysipelothrix rhusiopathiae;* Clostridium species, in particular *C. perfringens, C. tetani;* Enterobacter species, in particular *Enterobacter aerogenes,* Klebsiella species, in particular *Klebsiella pneumoniae,* Pasturella species, in particular *Pasturella multocida,* Bacteroides species; Fusobacterium species, in particular *Fusobacterium nucleatum;* Streptobacillus species, in particular *Streptobacillus moniliformis;* Treponema species, in particular *Treponema pertenue;* Leptospira; pathogenic Escherichia species; and Actinomyces species, in particular *Actinomyces israelli.*

23. The liposome, the mixture of liposomes or the liposomal composition of one of claims 1 to 22, wherein the first and the second adjuvant is selected from the group consisting of unmethylated DNA, in particular unmethylated DNA comprising CpG dinucleotides (CpG motif), in particular CpG ODN with phosphorothioate (PTO) backbone (CpG PTO ODN) or phosphodiester (PO) backbone (CpG PO ODN); bacterial products from the outer membrane of Gram-negative bacteria, in particular monophosphoryl lipid A (MPLA), lipopolysaccharides (LPS), muramyl dipeptides and derivatives thereof; synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, interleukin- (IL-)2, IL-6, IL-7, IL-18, type I and II, interferons, in particular interferon-gamma, TNF-alpha; 25-dihydroxyvitamin D3 (calcitriol); synthetic oligopeptides, in particular MHCII-presented peptides; gel-like precipitates of aluminum hydroxide (alum).

24. The liposome, the mixture of liposomes or the liposomal composition of claim 23, wherein the first and the second adjuvant stimulate different receptors and/or pathways within cells of the immune system.

25. The liposome, the mixture of liposomes or the liposomal composition of claim 24, wherein the first and the second adjuvant stimulate at least two receptors selected from the group consisting of type I cytokine receptors, type II cytokine receptors for, TNF receptors; and vitamin D receptor acting as transcription factor; and the Toll-like receptors 1 (TLR-1), TLR-2, TLR 3, TLR4, TLR5, TLR-6, TLR7 and TLR9.

26. The liposome, the mixture of liposomes or the liposomal composition of claim 25, wherein the first and the second adjuvant, which primarily stimulate different receptors are selected for:
a) type I cytokine receptors from the group consisting of GM-CSF, IL-2, IL-6 and IL-7;
b) type II cytokine receptors from the group consisting of IFN-α/β and IFN-γ;
c) TNF receptors from the group consisting of TNF-α and CD40 ligand;
d) vitamin D receptor from the group consisting of calcitriol;
e) TLR-1 from the group consisting of tri-acyl lipopeptides from bacteria and mycobacteria and soluble factors from Neisseria meningitides;
f) TLR-2 from the group consisting of lipopeptides, in particular Pam₃Cys, lipoarabinomannan from mycobacteria, peptidoglycan, zymosan and heat shock proteins (HSPs), in particular HSP70, ; lipoteichoic acid from gram-positive bacteria, phenol-soluble modulin from Staphylococcus species., in particular *Staphylococcus epidermidis,* glycoinositolphospholipids from Trypanosoma species, in particular *Trypanosoma cruzi,* glycolipids from Treponema maltophilum, porins from Neisseria, atyptical LPS from Leptospira species, in particular *Leptospira interrogans* and Porphyromonas species, in particular *Porphyromonas gingivalis;*
g) TLR-3 from the group consisting of viral double-stranded RNA and poly dI:dC;
h) TLR-4 from the group consisting of LPS from gram-negative bacteria and its derivatives, in particular monophosphoryl lipid (MPLA), HSPs in particular HSP60 and HSP70, Taxol, fusion proteins of RSV, envelope protein of MMTV, fibronectin and fragments thereof, oligosaccharides of hyaluronic acid, polysaccharide fragments of heparan sulfate and fibrinogen;
i) TLR-5 from the group consisting of bacterial flagellin;
j) TLR-6 from the group consisting of di-acyl lipopeptides from mycoplasma;
k) TLR-7 from the group consisting of imidazoquinoline, loxoribine and bropirimine; and
l) TLR-9 from the group consisting of unmethylated DNA, in particular CpG-DNA; unmethylated phosphorothioate (PTO) oligonucleotides, in particular CpG-PTO oligonucleotides.

27. The liposome, the mixture of liposomes or the liposomal composition of one of claims 1 to 26, wherein a targeting moiety is attached to the liposome.

28. A method for producing the liposome, the mixture of liposomes or the liposomal composition of one of claims 1 to 27, wherein the method of producing the liposome, comprises the steps of:
a) forming a suspension of at least one lipid, one or more therapeutic agent and optionally a first and/or a second adjuvant in a liquid medium and
b) homogenizing the suspension.

29. A liposome produced by the method of claim 28.

30. Use of a liposome, the mixture of liposomes or the liposomal composition of one of claims 1 to 26 or claim 28 for the production of a medicament for the prevention or therapy of proliferative diseases, infectious diseases, vascular diseases, rheumatoid diseases, inflammatory diseases, immune diseases, and allergies.

31. The use of claim 30, wherein the proliferative disease is selected from the group consisting of carcinomas of the gastrointestinal or colorectal tract, liver, pancreas, kidney, bladder, prostate, endometrium, ovary, testes, melanoma, dysplastic oral mucosa, invasive oral cancers, small cell and non-small cell lung carcinomas, hormone-dependent breast cancers, hormone independent breast cancers, transitional and squamous cell cancers, neurological malignancies including neuroblastoma, gliomas, astrocytomas, osteosarcomas, soft tissue sarcomas, hemangioamas, endocrinological tumors, hematologic neoplasias including leukemias, lymphomas, and other myeloproliferative and lymphoproliferative diseases, carcinomas in situ, hyperplastic lesions, adenomas, fibromas, histiocytosis, chronic inflammatory proliferative diseases, vascular proliferative diseases and virus-induced proliferative diseases.

32. The use of claims 30 or 31, wherein an adjuvant and/or a cytokine is (are) administered prior, simultaneously or after administration of the liposome or liposomal composition.

33. The use of claim 32, wherein the adjuvant is selected from the group of adjuvants consisting of unmethylated DNA, in particular unmethylated DNA comprising CpG dinucleotides, in particular CpG PTO ODN or CpG PO ODN; alum; bacterial products from the outer membrane of Gram-negative bacteria, in particular MPLA, LPS, muramyl dipeptides and derivatives thereof; synthetic lipopeptide derivatives, in particular Pam₃Cys; lipoarabinomannan; peptidoglycan; zymosan; HSP, in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, IL-2, IL-6, IL-7, IL-18, type I and II, interferons, in particular interferon-gamma, TNF-alpha; oil in water emulsions, in particular MF59 consisting of squalene; Tween 80 and Span 85 and QS-21, non-ionic block polymers, in particular Poloxamer 401, saponins and derivatives thereof; polyphosphazene; BAY R1005, calcitriol; DHEA; [MDP(Gln)-OMe; murapalmitine; polymers of lactic and/or glycolic acid; polymethyl methacrylate; sorbitan trioleate; squalane; stearyl tyrosine; squalene; theramide, synthetic oligopeptides, in particular peptides presented by MHC-class II.
